# EUROPEAN PATENT APPLICATION

(11) **EP 1 090 658 A1**
(43) Date of publication of application: **11.04.2001**
(21) Application number: 00308783.0
(22) Date of filing: 05.10.2000
(51) Int. Cl.: A61N 7/02

(54) **Ultrasonic medical treatment apparatus**

(30) Priority: 26.07.2000 US 625803; 28.01.2000 US 178901 P; 05.10.1999 US 157824 P
(71) Applicant: OmniSonics Medical Technologies, Wilmington, MA 01887 (US)
(72) Inventor: Rabiner, Robert A., Massachusetts 01864 (US); Hare, Brad A., Massachusetts 01824 (US); Fischer, David M., Massachusetts 02451 (US); Levine, Andy, Massachusetts 02159 (US); Meade, John, Massachusetts 01756 (US)
(74) Representative: Baverstock, Michael George Douglas

(57) **Abstract**

A method and apparatus for treating tissue using ultrasonic energy. The method and apparatus has particular application in debulking/removing prostatic tissue in a non-thermal manner in a patient suffering from benign prostatic hyperplasia (BPH). The apparatus is designed to have as small a cross-sectional profile as possible, therefore allowing the apparatus to be used in a minimally-invasive manner. As a result, the apparatus can be used in both surgical and out-patient treatment of BPH with minimal post-operative complications and minimal damage to areas other than the area of treatment. An ultrasonic probe may include aspiration channels on its outer surface. An aspiration sheath may surround the ultrasonic probe, such that the location of an aspiration port may be varied axially relative to the ultrasonic tip. The method of treatment of the present invention may be performed perineally, transurethrally, or urethrally. The ultrasonic probe of the present invention may be inserted into the prostatic capsule in a minimally-invasive manner, such that the bulked prostatic tissue is directly debulked in a non-thermal manner. As a result, the temperature at the treatment site may remain within ± 7° C of normal body temperature, so as to reduce necrosis and residual tissue damage. The ultrasonic probe is designed to vibrate in a direction transverse to a longitudinal axis of the probe. The transverse mode of operation is more efficient than the longitudinal mode of operation.

## Description

### Background Information

### Field of the Invention

The present invention relates to a method and an apparatus for medical treatment. In particular, the present invention relates to a method and apparatus for debulking the prostate by applying ultrasonic energy to prostatic tissue.

### Description of the Prior Art

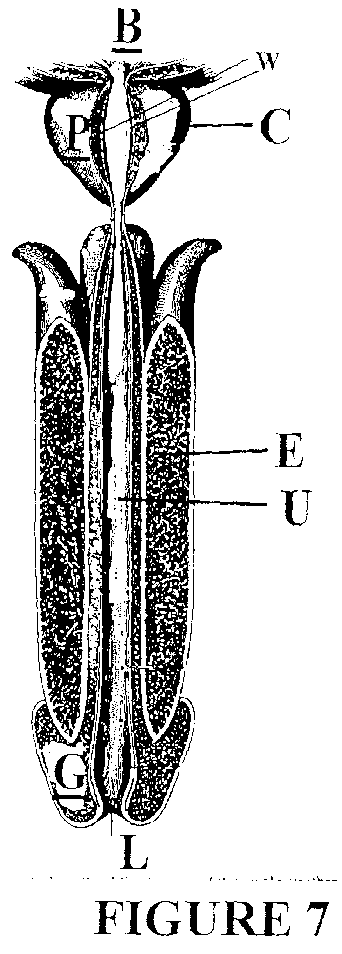

As shown in Figure 7, the prostate **P** is a fibrous muscular gland that lies immediately below the bladder **B** in the male. The normal prostate **P** is approximately the size of a walnut. The prostate **P** contains prostatic tissue in its interior, and is surrounded by a prostatic capsule **C**. The prostatic capsule **C**, in turn, surrounds the urethra **U**. The prostate **P** is surrounded by the prostatic capsule **C** and surrounds the urethra **U** of the penis **E** in the area of the bladder **B**. The urethral lumen **L** is located in the area of the glans **G**.

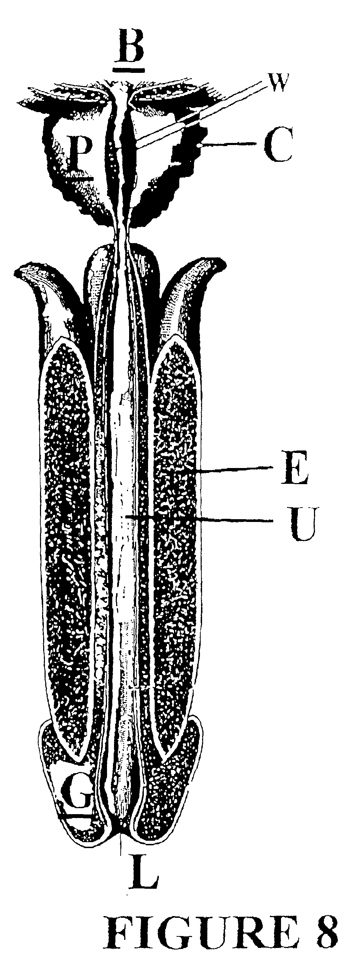

Usually, in approximately the fourth decade of life, the prostate **P** begins to enlarge and causes a condition called benign prostatic hyperplasia (BPH). In BPH, benign nodules grow in the prostatic tissue. The growth of these nodules causes the prostate **P** to push against the walls **W** of the tube-like urethra **U** that empties the bladder **B** and passes through the center of the prostate **P**. As a result, the urethra **U** can become restricted by the urethral walls **W** closing in towards one another. Figure 7 shows a normal prostate **P** in which BPH does not exist; Figure 8 shows a prostate **P** which is subject to BPH. As can be seen in Figure 8, the prostatic tissue **P** in the prostatic capsule **C** of a patient with BPH has expanded such that it pushes the wall **W** of the urethra **U** inwardly, partially obstructing the urethra **U** and also pushing on the prostatic capsule **C**. In the patient without BPH, the prostate **P** is reduced in size and does not cause the wall **W** of the urethra **U** to obstruct flow through the urethra **U**.

This growth of prostatic tissue can obstruct the flow of urine through the urethra U. As a result, men begin to experience problems with urination such as increased frequency, urgency to void, incomplete emptying of the bladder **B**, and burning or pain during urination. A delay in treatment can have serious consequences, including complete obstruction (acute retention of body waste or urine), loss of bladder functions, and in extreme cases, kidney failure The symptoms of BPH can be debilitating and can significantly alter the sufferer's quality of life. At present, BPH is usually treated by the surgical removal of the prostate tissue, or surgical reduction or destruction of urethral wall **W** which is compressed into and obstructs the urethra **U**. These surgical solutions, however, are a time consuming and expensive procedures, and carry with them significant post-procedure complications.

There are a number of known techniques for treating BPH. The first technique is trans-urethral resection of the prostate ("TURP"). TURF is the most common technique used today to treat BPH. In TURP, the urethra and the prostatic tissue is surgically removed. TURP entails significant possible side-effects, such as incontinence, impotence, sepsis, and bladder rupture, as well as many other problems attendant with a surgical technique and attendant with the complete removal of the prostate.

A second technique for treatment of BPH is trans-urethral needle ablation ("TUNA") In TUNA, a series of needles are inserted trans-urethrally into the prostate. These needles are then activated to produce radio-frequency (RF) energy into the prostate such that an intra-prostatic temperature of 90°-100° C is achieved (normal body temperature being approximately 37° C). This RF energy thermally destroys, or necroses, the prostatic tissue within the prostatic capsule. TUNA also has several disadvantages. Because TUNA uses thermal destruction, or necrosis, it results in edema or swelling, and therefore normally requires post-procedure catherization, to allow voiding until the edema subsides. Additionally, the thermal destruction of tissue is an inefficient technique for treating hyperplasic tissue, because tissue is not removed, rather it shrinks.

A third technique of treatment of BPH involves the use of microwaves and is sometimes referred to as TARGUS. In this technique, a urethral probe is directed to the area of the prostate, and thereafter emits microwave energy to thermally destroy, or necrose prostatic tissue. Like TUNA, it is desirable in the microwave technique to achieve an elevated temperature of 90°-100° C, so as to necrose, and possibly shrink, the treated tissue. Because the microwave energy is not targeted, this technique can destroy surrounding tissue, with adverse effects. Furthermore, this technique, like TUNA, relies on thermal destruction or necrosis, and therefore does not remove tissue. The thermal energy of the microwaves may also act to thermally destroy the tissue behind the urethra, rather than the prostate tissue itself, resulting in loss of elasticity in the urethra, with long-term complications such as incontinence land possible retrograde ejaculation.

A fourth technique for treatment of BPH is high-intensity focused ultrasound ("HIFU"). In HIFU, ultrasound energy is focused to the area of the prostate, in order to elevate the temperature of the prostatic tissue to 80°- 100° C, to thereby thermally destroy or necrose the prostatic tissue. Like TUNA and TARGUS, HIFU is a thermal technique, which can result in edema or swelling (and resulting in the potential need for catherization), and also can be non-site-specific, and therefore can potentially damage non-prostatic tissue.

A fifth technique for the treatment of BPH, which can entail a number of different mechanisms for tissue destruction, involves inserting a probe having a thermal energy source into the urethra. The probe can have at one end a coiled electrical loop, or a laser, which is passed up the urethra **U** to the area of the prostate **P** in the area of the sphincter of the bladder **B**. The end of the probe is then activated, so that it heats up the adjacent tissue to a temperature of 80°- 100° C. The probe is pulled in an outward direction from the urethra. The probe end heats up and thermally destroys or necroses the portion of the urethral tissue of the urethral wall which is in the area of the urethral obstruction. There are a number of complications which are the result of the use of this technique to threat BPH. Because the probe end passes along and removes urethral wall **W** tissue, and potentially removes the sphincter, incontinence may result. The application of thermal energy in the area of the bladder sphincter can also damage that sphincter, resulting in incontinence or retrograde ejaculation. Furthermore, the thermal destruction of tissue results in temporary edema or swelling in the urethra, which requires catherization, and attendant discomfort, to the patient.

One experimental technique for the treatment of BPH, is described in Krawitt et al., "Ultrasonic Aspiration of the Prostate, Bladder Tumors and Stones, *Urology,* 30:6 (1987), pp. 578-580. The technique for treatment of BPH described in that paper involved inserting a ultrasonic probe, normally designed for orthopaedic surgery, inwardly through the urethra. As the probe was inserted, the ultrasonic tip was activated, such that the ultrasonic energy destroyed the urethral wall tissue extending into and blocking the urethra that was directly in front of the tip of the probe. This technique involved at least two disadvantages. First, because the probe used was not intended for urethral use, the probe required that it be pushed inwardly during treatment, instead of the preferred technique used in other procedures whereby the probe is pulled outwardly during treatment. As a result, the surgeon did not have the opportunity to view the probe tip, and there was no way to tell where the tip was located along the urethra. It therefore was likely that the probe would be extended too far, thereby damaging the bladder sphincter and resulting in permanent incontinence and retrograde ejaculation This is because the ultrasonic probe used in the Krawitt et al. tip was large and rigid, and could only treat tissue directly in front of the tip during insertion, which was urethral wall **W** tissue. The Krawitt et al. technique, however, did have the advantage that it did not thermally destroy or necrose the tissue, and there was a reduction in edema. Another disadvantage of the Krawitt et al. technique is that the destruction of urethral wall tissue similar to other surgical modalities resulted in disadvantageous long-term side effects to the patient. In each of the techniques using a urethral probe, however, a significant disadvantage is that the probe may only destroy tissue directly in front of or immediately adjacent the tip, and therefore - when treating BPH - results in the destruction of both urethral tissue and prostatic tissue

One disadvantage of all of the prior art techniques described above is that the equipment necessary for preforming the procedures is generally expensive

U.S. Patent No. 4,867,141 discloses a medical treatment apparatus which utilizes ultrasonic energy for medical treatment, particularly to break up a stone formed in a living body. An endoscopic channel is used to insert a portion of the apparatus into a body cavity, where an ultrasonic transmission member is used to transmit ultrasonic vibrations to the stone which is in contact with the distal end of the apparatus. A perfusion liquid is supplied to the area of the stone as the stone is being broken up by mechanical ultrasonic vibrations. This perfusion liquid is sectioned away from the area of the stone. As a result of the suction, the perfusion liquid and broken pieces of the stone are drained away from the body cavity. The apparatus of that patent is configured with an ultrasonic transmission member which is aligned and coaxial with the central axis of the probe, and therefore is effective in treating conditions -- such as stones -- where the irregularity or condition to be removed is aligned with the body vessel through which the endoscope passes. The device of that patent is used on non-hydrated calcified tissue, and uses direct mechanical vibration of the calcified tissue in order to result in tissue fracture and destruction.

U.S. Patent No. 5,176,677 discloses an endoscopic ultrasonic rotary electro-cauterizing aspirator. The background section of that patent includes some discussion of medical literature relating to prostatectomies, and in particular the Krawitt et al technique, in which a gland can be removed using ultrasonic treatment without effecting to the prostatic capsule. However, the apparatus shown in that patent is disclosed as being useful for arthroscopic surgery. The apparatus shown in that patent includes a feature for providing irrigating fluid to the tip of the ultrasonic probe, as well as mechanisms for aspirating the area around the tip. The aspiration and irrigation features of that invention require individual passageways coaxial with the ultrasonic working tip, each connected to a source of pressurized fluid, for irrigation, or to a source of reduced pressure, for aspiration. The apparatus of that invention also includes other features adjacent the tip, such as an insulated hood for removing obstructions, and a telescopic viewing apparatus. The irrigation, aspiration, insulated hood and telescopic viewing apparatuses all increase the cross-sectional profile of the apparatus. The design of that apparatus also is such that it may only treat areas which are directly axially in front of the ultrasonic probe, and therefore which are axially aligned with the lumen or incision through which the probe is inserted.

Various other patents show apparatuses which use ultrasonic energy to fragment or transform body tissue. U. S. Patent Nos. 5,112,300; 5,180,363; 4,989,583; 4,931,047; 4,922,902 and 3,805,787 each show ultrasonic treatment apparatuses for use in treating various medical conditions. In each of these patents, some mechanism is shown for providing irrigation and/or aspiration in the area where the ultrasonic treatment is being performed In each of these patents, however, the mechanisms for irrigation or aspiration are structured such that they increase the overall cross-sectional profile of the instrument. In addition, in each of those patents, the irrigation and aspiration ports are a fixed distance from one another, which may not be varied.

"Ultrasonic processing," as used in the prior art for, inter alia, orthopaedic surgery, is a technique wherein a body -- either liquid or solid - is, in effect, "blasted" by ultrasonic energy. In ultrasonic processing, the ultrasonic energy produced by the ultrasonic vibrator influences water molecules found within the body tissue. The ultrasonic energy is in the form of very intense sound vibrations at a very high frequency. These intense, high-frequency sound vibrations results in powerful chemical and physical reactions in the water molecules within the body tissue. The reactions in the water molecules ultimately results in a process called "cavitation," which can be thought of as a form of cold (i.e., non-thermal) boiling of the water in the body tissue, wherein there is a rapid creation and collapse of numerous microscopic bubbles in the water.

The result of cavitation in water is a "breaking" of that fluid. The rapid vibrations in water caused by the application of ultrasonic energy to the water and the resultant cavitation can cause fatigue in the water molecules which will break bonds between the water molecules. The result is that the water changes from a liquid form into a gaseous form, i.e., converts into steam, but this conversion is done without the need for application of thermal energy to the water. The result is a "cold boiling" of the water.

When a steam bubble is created in a cold liquid, such as upon the application of ultrasonic energy to water, the steam will condense because it is surrounded by a cold liquid. As a result, a void or cavity is created. The surrounding water molecules rush in to fill that cavity, when they reach the center of the cavity, they collide with each other with great force. This process is called cavitation. Cavitation is a known phenomenon which results in shock waves running outward from the collapsed bubble. The shock waves caused by cavitation can wear away or destroy material, and are know to wear away metal at the edges of an outboard motor propeller.

Ultrasonic processing or ultrasonics is the application of sound at extremely high intensity and high frequency (normally above human hearing, 20kHz - i.e. 20,000 cycles per second - and above) so as to result in material changes. Ultrasonics are used in a number of different applications in order to change a variety of different materials. Ultrasonics accelerates both physical and chemical reactions in the materials to which ultrasonic energy is applied and these reactions, among many other things, are accomplished largely due to the action of cavitation. There are more actions inherent in bubble collapse which are of significance. As used herein, the term "bubble" refers to a space within a liquid which contains a gas or vapor. However, after that gas or vapor condenses, there is still a void or cavity in that space until an implosion occurs. Therefore the term "bubble," as used herein, also refers to the void or cavity.

One description of the manner in which cavitation is used in medical applications has been provided by Professor Lawrence Crum of the Applied Physics Laboratory at the University of Washington in Seattle, and can be found at the website:<http://pluto.apl.washington.edu/harlett2/artgwww/acoustic/medical medical.html>. Professor Crum, writing about lithotripsy - in which a kidney stone is broken with ultrasonic energy -- has stated that "[w]hen pressure surrounding a bubble falls below the vapor pressure of the liquid, the bubble fills with vapor and grows explosively The bubble collapses violently when pressure returns. If the collapse occurs near a boundary, such as [a] targeted kidney stone, a high velocity liquid jet is formed that impacts the boundary with great force. These extremely violent processes are thought to play a major role in stone destruction and associated tissue damage.

In addition to erosion or ablation of surfaces by the jet, cavitation causes many other actions. Notable among these in a purely physical sense is the action of intense shock fronts generated by imploding cavitation bubbles against kidney stones (lithotripsy), gall stones, tumors, and other intrusions in the body. Some of this action can also be accomplished by direct impact of a vibrating ultrasonic tool tip, but no (or minimal) cavitation is involved.

### Summary of the Invention

The present invention is directed to a method and an apparatus for treating tissue using ultrasonic energy. The present invention has particular application in debulking prostatic tissue in a patient suffering from BPH. The apparatus of the present invention is designed to have a small cross-sectional profile, therefore allowing the apparatus to be used in a minimally-invasive manner. As a result, the present invention is advantageous in that it can be used in both traditional surgical sites and out-patient treatment of BPH with minimal postoperative complications and minimal damage to areas other than the area of treatment. The present invention therefore provides distinct advantages over the prior art in the treatment of BPH, and therefore provides an improved method of treating BPH. It is to be understood, however, that although the present invention is designed for treatment of BPH, its small cross-sectional profile makes it useful for treatment of any condition wherein minimally invasive techniques are advantageous and reduce post-surgical complications, and the present invention is therefore not limited to the treatment of BPH. For example, the removal of polyps is possible using the invention as well as the removal of fibroids which are common in gynecology.

One particularly advantageous aspect of the present invention is that it is adapted for use wherein the probe is inserted into the urethra so that the probe may be inserted into the prostate by penetrating the urethral wall and advancing the probe tip to the interior of the prostatic capsule The probe of the present invention is specifically designed to have a minimal cross-section, particularly for use for prostate debulking, thereby minimizing post-procedure complications and discomfort to the patient because larger sizes are more difficult to insert within the urethra and are obviously uncomfortable. One way in which the present invention allows the cross-sectional profile of the probe to be minimized is by allowing aspiration to occur through grooves or channels on the outer surface of the probe. In this way, there is no need for an additional tubular aspiration sheath to be inserted into the urethra, to thereby provide a aspirating path. Instead, the aspiration probe sheath can be located completely outside the prostate - in the area of the lumen of the glans - thereby allowing full aspiration to occur without the need for a separate aspiration channel- creating sheath to be inserted into the urethra The aspirating lumen or opening can therefore be varied in its distance from the probe tip and any irrigation ports, such that the aspirating lumen or opening can be adjustably located at the point where the aspiration tube meets the urethra wall at the point of the probe insertion. As a result, the aspiration mechanism, other than the aspirating channels on the probe, may be located entirely outside of the prostate, reducing the overall area affected by the apparatus and decreasing tissue damage and edema. Another advantage of the use of channels for aspiration is that they are easier to contact with the materials to be aspirated and to remain clear than an internal passage for aspiration, and they also are less prone to blockage than an internal aspiration passage.

The present invention also allows treatment of the prostate through a perineal incision. The perineum is the area of the male between the scrotum and the rectum, and that area is very near the prostate. As a result, a small surgical incision can be made in the perineum to gain access to the prostate with minimal tissue disturbance. By using the minimally-invasive ultrasonic probe of the present invention, aperineal incision can be particularly small, while still gaining good access to the prostate. As a result, a perineal technique of the present invention is advantageous in that it can result in minimal surgical complications, while still allowing significant access to prostatic tissue for treatment.

In the apparatus of the present invention, the irrigation passage is preferably centrally located in the probe. The terminus or termini of the irrigation passage can be located at the tip of the probe, or can be lateral to the probe tip. When the terminus or termini of the irrigation passage are lateral to the probe tip, thereby exiting on the probe sides, the area of effect of the ultrasonic energy is increased.

A significant advantage of the technique of the present invention is that it physically destroys and removes bulked prostatic tissue through the mechanism of cavitation, which is non-thermal. The removal of tissue by cavitation also provides the ability to remove large volumes of tissue without large holes in the urethra with a small diameter probe. Accordingly, because of the use of cavitation as the mechanism for destroying tissue, together with the use of irrigation and aspiration, the method and apparatus of the present invention can destroy and remove tissue within a range of temperatures of ±7° C from normal body temperature, and therefore complications attendant with the use of thermal destruction or necrosis of tissue - such as swelling or edema, as well as loss of elasticity are avoided. The present invention is particularly advantageous because it non-thermally destroys and removes prostatic tissue reducing the symptoms of BPH at the time of the procedure, and therefore provides many advantages over prior art techniques, all of which either rely on surgical removal of the prostatic tissue and urethra, or thermal destruction or necrosis of prostatic tissue without tissue removal. The present invention is the only method for removing tissue without destroying the urethra.

The method and apparatus may be used advantageously in each of three different techniques. One technique with which the present invention may be used is in an outpatient setting and transurethrally. The physician administers a local anesthesia such as lidocaine jelly and/or oral sedative to the patient prior to treatment. After administering the local anesthetic or oral sedative, the apparatus of the present invention may be inserted transurethrally - i.e., through the patient's urethra - until the tip of the ultrasonic probe penetrates the prostatic capsule and is in contact with the bulked prostatic tissue. The ultrasonic vibrator may then be activated to produce ultrasonic energy at or along the probe tip, to thereby destroy and aspirate out portions of the enlarged tissue by cavitation. Another technique with which the present invention may be used is in a standard hospital operating room, where the patient is under general anesthesia. After the patient has been anesthetized, a percutaneous perineal incision is made (a skin incision between the scrotum and rectum), and the ultrasonic probe of the present invention is inserted through the incision, surgically delivered through the prostatic capsule, and then inserted into the prostate. Thereafter, the ultrasonic vibrator is activated so that the ultrasonic probe tip applies ultrasonic energy to the prostatic tissue, thereby destroying it via cavitation. The preferred technique with which the present invention may be used is transurethrally, by inserting the probe of the present invention through the urethral lumen of the glans and up the urethra until the ultrasonic tip is adjacent the enlarged prostatic tissue which causes the urethral wall to close or obstruct the urethra. Thereafter, the ultrasonic vibrator is activated, and the ultrasonic probe tip is inserted through the urethral wall tissue, and into the enlarged and bulked prostatic tissue. Thereafter, the probe is moved through the prostatic tissue, destroying, debulking, and removing via aspiration that tissue that was affected by cavitation, as the tip is moved into various locations in the prostatic tissue In each technique for use of the present invention, the low cross-sectional profile of the present invention reduces or eliminates any damage adjacent to any tissue outside of the enlarged prostatic tissue which is treated, and greatly reduces surgical, procedural, and post-operative complications.

The probe of the present invention, because of its significantly reduced cross-sectional profile, can be made to be flexible or bendable. The probe can be of a cross-section sufficiently small that the material of the probe is bendable through a wide range of articulation angles. The probe may housed in an articulated catheter or sheath, which catheter or sheath can be fabricated to be bendable or articulated. As a result, the present invention can be adapted to allow for the probe to activate and treat areas of the body which are not axially aligned with the channel or lumen through which the probe is inserted. As a result, the probe of the present invention has many more surgical applications, and it can be used to treat a greater variety of tissue, than prior art devices and methods. The articulation described above where the probe of the present invention is bendable allows for a "windshield wiper" action of the probe, in conjunction with the transverse cavitation mode of operation, such that an arc of tissue can be treated, greatly increasing the area of effect of the treatment through an incision.

The probe of the present invention is particularly useful in a treatment technique in which the treated area may be imaged by ultrasound imaging, in particular color ultrasound. The vibrating action of the probe echogenically produces a pronounced and bright image on the ultrasound, and therefore is readily viewable and discernable as the probe (as opposed to surrounding tissue) by the surgeon or physician, greatly increasing the ease of use and effectiveness of treatment. The ultrasound transducer probe is in the treatment of BPH preferably inserted into the rectum of the patient. In other procedures, the transducer may be located on the surface of the skin.

The probe of the present invention can also be designed to have a clamping and cutting feature, which is useful for surgical procedures in which the treatment area dangles or is loose in the body lumen or hollow organ of the patient. This embodiment of the probe is particularly useful for gynecological treatments, A sheath or catheter surrounding the probe includes a lateral opening, into which dangling or loose treatment areas can be captured and cut by a reciprocating probe tip.

The probe of the present invention is particularly amenable, because of its small size, to the use of a flexible fiberoptic viewing device. The device preferably includes a fiberoptic cable which is inserted along with the ultrasonic probe - either attached to the probe or separate from the probe - and which cable is connected to a fiberoptic viewing eyepiece which is not connected to the handle or other equipment of the ultrasonic mechanism. In this way, manipulation of the viewing system is reduced to a minimum. The fiberoptic cable preferably includes a central optical viewing cable surrounded by illumination fibers. The fiberoptic cable may be located inside of a flexible sheath portion of the device, located on top of the flexible sheath, or may be located on top of a rigid sheath of the device.

The method of the present invention, because it uses ultrasonic energy in a cavitational and transverse mode which does not provide thermal effect, is localized in effect, does not affect tissue other than that of the specific prostatic lobes being treated, and because it is of a reduced cross-sectional profile so as to prevent damage to the surrounding tissue during insertion, treatment, and removal, reduces pr eliminates tissue damage, irritation and swelling in the patient. As a result, the method of the present invention is particularly advantageous because it may reduce or eliminate the need for post-procedure catheterization or stenting of the patient both due to the minimal size and the lack of thermal effect. However, if after the procedure the patient is unable to urinate due to temporary swelling or irritation of the urethra, a temporary catheter or stent may be inserted into the patient's urethra.

The method of the present invention presents advantages over prior treatment regimens for BPH in that it results in immediate debulking of the prostate at the time of the procedure while sparing the urethra. Prior art treatments do not result in actual debulking of prostatic tissue, because they either: 1) necrose and otherwise destroy urethral wall tissue in the affected area; and/or 2) thermally destroy prostatic tissue through necrosis, which does not debulk tissue, but instead merely kills and then due to tissue death the tissue shrinks after a period of time. From a patient perspective, due to the immediate debulking of the prostate in the present invention, the restriction/compression on the urethra is almost immediately reduced, allowing for unrestricted urination by the patient almost immediately without catheterization. Due to the decreased size requirements of the present device, the edema that is caused may also be decreased, as smaller diameter instruments do not stretch the urethra, causing less damage. The present invention also provides advantages in that it is a minimally invasive procedure, which may be -- and is preferably - an outpatient procedure. Therefore, the method of tissue reduction of the present invention is a minimally invasive, out-patient, procedure that provides for the immediate relief of the symptoms of BPH. The other out-patient treatments now in use for BPH each require several weeks before the affected and necrotic tissue is reduced in volume, thereby resolving the symptoms because of their failure to actually remove tissue and because of the ancillary damage due to size and heat. Therefore, the present invention is advantageous over techniques currently in use in that the present method provides immediate relief to the patient of the symptoms of BPH. The present invention also provides a faster procedure than the prior art.

The present invention also provides advantages to the physician rendering the treatment. The present invention is safer for the physician, because the components of the apparatus and method of the present invention deliver only ultrasonic energy to the surgical site, and as ultrasonic energy's affect on tissue is based on tissue planes and their hydration levels, tactile feel to the surgeon is preserved. Similarly, due to tissue differentiation, the apparatus of the present invention directs the cavitation only into the interior of the prostate and the prostatic tissue, and as a result protects the prostatic capsule, bladder sphincter and bladder neck, thereby reducing the risk of unintended thermal damage to surrounding structures. Each of these non-prostatic tissue areas have different hydration levels relative to prostatic tissue, and therefore may not be affected by the ultrasonic energy to the same extent. Furthermore, the area in which the cavitation is effective in the apparatus of the present invention is an area on the order of 1-2 mm circumferentially around the ultrasonic probe without subsequent spread of energy or tissue affect. As a result, the present invention allows a much higher degree of control of the affected area than prior art techniques. In other procedures where the control of tissue damage of the present invention does not exist, the urethra and other structures can be damaged or destroyed by the thermal spread or other energy egress intended for the prostatic tissue, causing significant patient discomfort and unintended complications such as urinary incontinence, impotence and retrograde ejaculation. Because the present invention results in the destruction of very little or no compliant urethral wall (save for the penetration by a small, less than 1 mm diameter probe) or prostatic capsule wall tissue, in contrast to the prior art, these post-procedure complications may be significantly reduced

In the application of ultrasonic energy towards the controlled destruction and removal of tissues within a cavity -- specifically where the ultrasonic probe is to be used in a manner so that it drills and/or creates a void in a cavity in an organ, where the tip of the probe is inserted substantially beneath the surface of the tissue - there needs to some mechanism to remove tissue and particulate from this area caused by the ultrasonic drilling or tissue removal. Traditional methods for removal of materials and exudate is through the use an aspiration mechanism which provides suction through a suction passage which is part of the probe. These traditional methods use irrigation flow of a fluid which is injected into the site where the procedure is being conducted, in conjunction with aspirations to remove the tissue from the surgical site. As discussed above, however, providing both irrigation and aspiration to the surgical site has, in prior art apparatuses, resulted in a probe of relatively large cross-sectional profile. Therefore, the treatment apparatus is substantially larger than the ultrasonic probe needed for conducting the procedure. These prior art apparatuses used concentric tubes wherein the irrigant is normally provide through a central core of the probe and the aspirant is provided an outer concentric tube and lumen.

Prior art apparatuses also maintain a strict orientation of the suction and the irrigation mechanism, such that the inner and outer lumens for irrigation and aspiration remain in a fixed position relative to one another, generally in a position closely adjacent the area of treatment. Thus, the irrigation lumen does not extend beyond the suction lumen (i.e., there is no movement of the lumens relative to one another) and the suction is designed to pick up any fluid and/or tissue remnants within the defined distance between the two lumens. The present invention uses grooves or channels on the outside of the probe for aspiration and irrigation through the control portion of the probe. The irrigation exits the central portion on the sides of the probe. This allows for the distance between the irrigation and aspiration lumens to be varied, thereby reducing the cross-sectional profile of the instrument inserted in to the patient's body. The reduced cross-sectional area allows easy insertion through the urethra. In the present invention, a axially movable aspirating catheter or sheath may move along the length of the probe, to thereby vary the position of the aspiration lumen relative to the probe tip and the irrigation lumen or lumens. This allows for the aspiration lumen, and the associated aspiration structure - except for the grooves or channels on the probe - to be located outside the patient's body.

Similarly, as the probe causes a void to be created within the tissue, it forms a pocket with only one entry point. Suction can be applied to this entry point, which is external to the area affected by ultrasonic energy, to aspirate material through the channel created by the probe and maintained patent by the probe.

In the application of the ultrasonic energy of the present invention the probe diameter is substantially smaller than that of traditional ultrasonic probes, and therefore is ideally designed for minimally invasive procedures The probe of the present invention is therefore designed to be used in areas of the body -- for example, the urethra - where a smaller probe is less invasive, and therefore less painful and the smaller size permits removal of tissue without incurring incremental tissue response, usually in the form of inflammation and swelling due to the size of the instrument inserted into the urethra, stretching the tissue to accommodate it, thereby causing the need for additional apparatuses, such as catheters, to be used to maintain the lumen of the organ open. The present invention therefore relates to the application of small diameter probes, which can be inserted into a small diameter body vessel to thereafter use a cavitational effect to remove tissue. In order to increase the area of treatment which is effective in the small probe of the present invention, the irrigation lumens can be transverse to the axis of the probe, i.e., can open on the sides of the probe body. In this way, the maximum area of the probe tip is used to provide ultrasonic treatment and cavitational energy.

The probes used with the present invention are shaped to allow easy insertion through the urethra, and so that they are not sharp so as to present the risk of tissue damage during insertion. The probes may include a taper that accommodates the insertion of the probe into the tissue through the application of the ultrasonic energy, wherein the velocity amplified through the transition from a larger mass to a smaller mass. The tapered shape of the probe, usually that of a cone, sphere, or hemisphere, is particularly useful in surgical applications as the tissue rent or tear caused by the ultrasonic drilling of the probe tip is formed in a manner where there are no tears or sharp comers that would cause the tear to expand beyond that of the initial penetration point. Similarly, the tapered shape of the probe minimizes the overall size of the penetration point through the tissue, as the ultrasonic energy is focused towards the distal end of the probe and diminishes in amplitude and frequency as the distance from the tip is increased. Because the probe has a relatively constant diameter, the tissue fits snugly around it.

One issue with the use of a probe in which the tissue fits tightly around the probe is that the close fitting tissue can cause the irrigant to be caught and stored within the organ or tissue, with a hydrostatic load being caused. This hydrostatic load, through the insertion of fluid within the tissue space, causes potential unwanted results, since the fluid becomes a vehicle for dissecting and distending the tissue planes. The dissection can lead to unwanted rupture of the vasculature, as well as connective tissues, nerves and the like. Similarly, distention of tissue and organs beyond that of traditional or normal size may have a deleterious effect on their normal and usual function, and may impede their function for periods well beyond the time of the procedure.

In order to negate the deleterious effects associated with the ingress of fluid, there needs to be a mechanism whereby the fluids and the particulate caused by the cavitation can be removed from the cavity, without an increase in diameter of the initial rent in the tissue, without the need for sophisticated pressure monitoring and without the need for secondary holes.

The present invention solves the problem of fluid ingress and retention by the application of grooves or channels on the surface of the ultrasonic probe, where the surface of the probe may be curved so as to form a continuous curve, where the grooves or channels are drilled into the surface of the probe, thereby lowering the profile of the grooves or channels. Where the grooves or channels start towards the proximal end of the probe (near the tip) and increase in depth and width as they extend towards the distal end of the probe, the effect of the grooves or channels is to draw away fluid and cavitated tissue in rapid fashion. In the present invention, it is preferable that two, three, four or a greater number of longitudinal grooves or channels extend from the tip towards the end of the probe. The use of a single or dual grooves or channels starting at the proximal end of the probe and traversing the diameter of the probe in a spiral fashion (similar to that of a screw) may also be used for the same effect in the present invention.

The purpose of these grooves or channels, and their particular shapes, is to allow the fluid and particulate a path to escape the cavity. The grooves or channels also allow the materials and the fluid to be flushed from deep within the tissue. The grooves or channels also for a continual exchange of fluids below the surface of the tissue to maintain a normal physiologic condition. This is in particular caused by providing an irrigation fluid to the cavitation site, creating a continual cavitation liquid media, which ensures extracellular water content, via a fluid exchange, so that the ultrasonic energy does not, upon the destruction of cellular matrix and the removal of intracellular fluid, cause any unwanted or undesired thermal heating.

The grooves or channels provide advantages over prior art aspiration configurations in several respects. First, the use of grooves or channels eliminates the need for an additional sheath surrounding the probe to create an aspiration passage. As a result, the cross-sectional profile of the instrument can be reduced, thereby reducing residual tissue damage and edema. Furthermore, the grooves or channels allow an aspiration sheath to be located outside of the passage or lumen to the treated area - such as the urethra.

Furthermore, because the grooves or passages are located on the outside of the probe, they are less likely to become obstructed or clogged, and furthermore, they are much easier to clean than internal aspiration passages.

The present invention, therefore, includes a number of advantageous features which make it particularly useful for minimally-invasive procedures. First, it allows for the application of ultrasonic energy to tissue, such as the prostatic capsule, via a small diameter probe that is able to traverse a vessel or opening, such as the urethra, and which can remove tissue through the action of cavitation on the tissue. The present invention therefore can create a cavity and debulk the prostate. Second, the present invention has the ability to make a small penetration into and through a body vessel, such as the urethra, or to create a small penetration in the body, such as in the perineal space between the scrotum and the anus, and thereafter allows the probe to be directed to tissue to be treated, such as bulked tissue in the prostate. Third, the present invention allows, through the action of cavitation and a movement by the surgeon of the ultrasonic tip throughout the treated area, the creation of a large cavity, so that the cavity is larger than the size of the probe. Fourth, the apparatus of the present invention may be designed with a stiffened outer member, within which a softer malleable or bendable member resides, which therefore allows the ability to insert a probe into the body, such as the urethra and prostate, without extreme torsion and bending on the anatomy. This malleability also allows the probe to be bent or articulated so that it can reach areas which are not axially aligned with the lumen or passage into which the probe is inserted. Fifth, the present invention allows the ability to remove tissue at the point of the procedure. Sixth, the present invention allows the ability to irrigate the cavitation site via the ultrasonic probe - both when the probe is rigid and when the probe is flexible and malleable. Seventh, the present invention allows the use of ultrasonic energy which is applied to tissue selectivity, because it uses energy in a frequency range - 20 kHz to 80 kHz - such that it imparts the energy specifically to hydrated (water-laden) tissues. As a result, there is little or no energy imparted to high-collagen connective tissue. This selectivity in the application of energy therefore spares those tissues which are important to retaining normal fluids, such as the tissue in the bladder neck and internal sphincter, and therefore prevents postprocedure complications such as retrograde ejaculation, incontinence, etc. which are specific to urologic applications. Eighth, the present invention allows the use of bending, flexing or articulated probe, which limits the amount of force placed upon the probe as it is advanced forwards, and therefore limits any force directed towards the prostatic capsule. In this way, the force applied on the probe is decreased through the bend, i.e., less pressure is imparted on the tip of the probe, thereby decreasing the potential for accidental penetration of tissue by the probe through pure physical force. That disadvantageous result might have occurred in a similar application of force using a straight probe into the prostatic capsule.

An important feature of the present invention is that it is non-thermal; in its effect on tissue. The use of a procedure in which heat or thermal energy is not used is important, and the present invention produces very low heat, and the excursion of heat is limited to the immediate area associated with the acoustic wave that is ablating the tissue The present invention, through the use of ultrasonic energy together with irrigation and aspiration, can produce treatment within a range of normal body temperature in the treated area of ±7° C. This is particularly advantageous in the treatment of BPH (as well as other applications where lack of thermal spread to health tissue is important - e.g. gynecology), as it can debulk prostatic tissue without necrosis, and without attendant thermal damage to surrounding tissue. In addition, this temperature range, because of aspects of the probe of the present invention, can be applied directly to the prostatic tissue, rather than to urethral tissue, which can result in complications. Furthermore, collateral irrigation in the form of extracellular fluid causes the residual tissue to remain within a narrow range around normal physiologic temperatures throughout the procedure of the present invention. Due to the lack of heat in the present invention, and the immediacy of the tissue removal process, the residual tissue exhibits remarkably little to no inflammatory response, thereby producing little edema (swelling) of the surrounding tissue. By virtue of the minimal swelling, the patient may be able to be discharged without the use and aid of either a catheter or a stent post procedure, or if such procedures are necessary, those procedures may be only for a minimal period of time Furthermore, prior art methods for remediation of BPH symptoms -- except for TURP, a traditional procedure which is extremely invasive and requires some degree of overnight hospitalization - thermally destroy the bulked prostatic tissue at a temperature of above 45 ° C, resulting in an exposure of the surrounding, non-prostatic, tissue to a lethal temperature This can result in destruction of prostatic and non-prostatic tissue, and furthermore that process, which relies on necrosis, requires 4 to 8 weeks for the tissue to be shrunken, but does not result in any volume of tissue being removed. Those disadvantages do not exist in the present invention

The present invention would be amenable to the subsequent use, after prostate debulking, of autologous and/or other tissue glues to be injected into the rent or cavity created by the procedure, to thereby close the tissue rent or cavity and glue it shut. The result of that procedure would be an even a faster decompression of the urethra, and less chance of post-procedure complications, as the rent in the tissue is closed.

Another advantageous feature of the present invention is that the ultrasonic probe can bend after insertion into a body vessel such as the urethra. That feature allows the ultrasonic tip to contact and cavitate tissue which is located on the wall of the vessel off axis, unlike prior art apparatuses in which the ultrasonic probe is rigid and can only contact tissue or physical abnormalities which are aligned with the body vessel or incision into which the probe is inserted. Therefore, in the present invention, the ultrasonic probe may be inserted into a relatively small diameter lumen or vessel, and thereafter bent at an angle (which angle may be relatively sharp) so as to penetrate tissue on the side of the lomen or vessel.

The ultrasonic tip of the present invention, because is uses cavitation as the mode of tissue removal, does not have to be sharp, because the tissue penetration is caused by cavitation, not by the physical shape of the probe. Therefore, the: tip can be smooth, making insertion less traumatic and less prone to residual tissue damage.

The ultrasonic energy to be applied to a particular treatment site is a function of the amplitude and frequency. In general, the throw rate or amplitude of the energy supplied by the apparatus of the present invention is in the range of 150 microns to 250 microns, and the frequency in the range of 20-80 kHz.

### Brief Description of the Drawings

Figure 1 is a side elevation view of handle of the ultrasonic treatment apparatus of the present invention;
Figure 2 is a perspective view of a first embodiment of the ultrasonic treatment apparatus of the present invention;
Figure 3 is a side elevation view of the embodiment of Figure 2;
Figure 4 is a perspective view of one embodiment of an ultrasonic tip of the present invention,
Figure 5 is a perspective view of a second embodiment of an ultrasonic tip of the present invention;
Figure 6 is a side elevation view of a second embodiment of an ultrasonic treatment apparatus of the present invention;
Figure 7 is cross sectional view through the urinary tract of a patient not suffering from BPH;
Figure 8 is cross sectional view through the urinary tract of a patient suffering from BPH;
Figure 9 is a cross-sectional view through the lower torso of a patient, showing a first step of a surgical procedure of the present invention;
Figure 10 is a cross-sectional view through the lower torso of a patient, showing a second step of a surgical procedure of the present invention;
Figure 11 is a radial cross-sectional view through an embodiment of an ultrasonic probe of the present invention;
Figure 12 is a partial perspective view of one embodiment of an ultrasonic treatment apparatus of the present invention;
Figure 13 is an axial cross-section of one embodiment of an ultrasonic treatment probe of the present invention;
Figure 14 is a cross-sectional view of a patient's urethra during treatment of BPH using one embodiment of the present invention; and
Figure 15 is a cross-sectional view of a patient's urethra during treatment of BPH using another embodiment of the present invention.
Figure 16 is a plan view of a right angle ultrasonic tip.
Figure 17 is a perspective view of a right angle ultrasonic tip.

### Detailed Description of the Invention

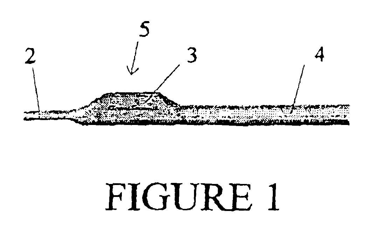

Figure 1 shows an embodiment of a handle 5 used with the present invention. The handle **5** is composed of an irrigation fitting or luer **2**, a grasping area **3**, and a probe fitting **4**. The irrigation fitting or luer **2** is configured for connection with a flexible tube which is in turn connected to a source of pressurized irrigating fluid, such as water. The grasping area **3** is shaped for grasping by the hand of the apparatus operator, such as a surgeon, and may include one or more trigger or button mechanisms for activating and deactivating various features of the apparatus, such as suction, irrigation, power, etc.

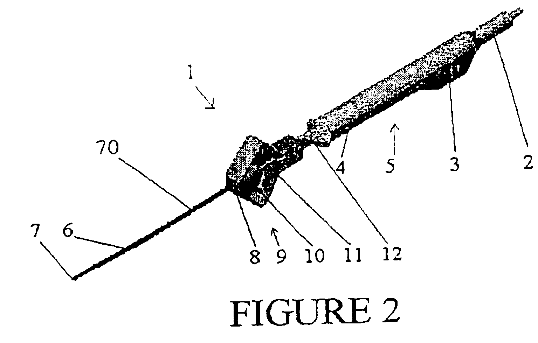

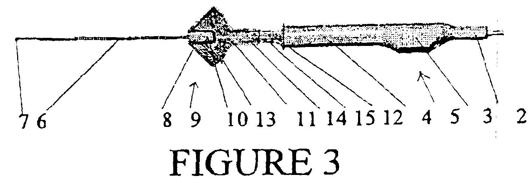

Figures 2 and 3 show an embodiment of the ultrasonic treatment apparatus **1** of the present invention, which includes the handle 5 shown in Figure 1. The ultrasonic treatment apparatus **1** includes an ultrasonic probe **6** with an ultrasonic probe up **7**. The ultrasonic probe **6** is axially movably mounted within an aspiration sheath or catheter **70**, so that the probe tip **7** may move axially inwardly and outwardly relative to the distal end of the aspiration sheath or catheter **70**. The ultrasonic probe **6** and aspiration sheath or catheter **70** are both mounted in an aspiration shroud **9**, which includes an aspiration shroud housing **8**. Within aspiration shroud housing **8** is an aspiration end **10** of aspiration sheath or catheter **70**,which transmits suction or negative pressure to the interior of aspiration sheath or catheter **70**. The aspiration end surrounds, and is sealed against, the ultrasonic transmission element **11** which extends to, and forms a proximal portion of, the ultrasonic probe **6**. The aspiration end **10** is connected an aspiration fitting or luer **13**. The aspiration fitting or luer **13** is configured for connection with a flexible tube which is in turn connected to a source of reduced pressure. The aspiration sheath is slidable relative to handle **5** and probe **6**, thereby allowing the distance between the ultrasonic tip **7** and the distal end of the aspiration sheath or catheter **70** to be varied. An actuation mechanism **12** may extend from the aspiration shroud **9** to the handle **5**, and is surrounded by suitable covers **14** and **15**.

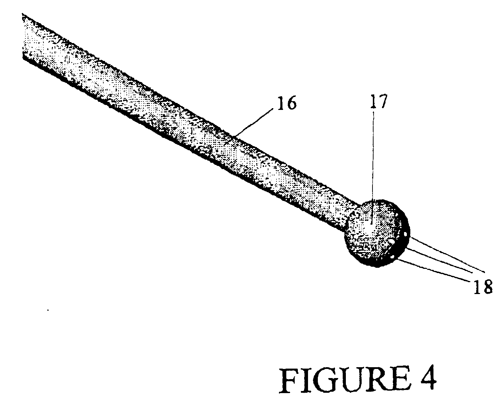

Figure 4 shows an embodiment of an ultrasonic probe **16** and ultrasonic probe tip **17** of the present invention. The body ofthe ultrasonic probe **16** in the embodiment of Figure **4** is preferably slightly tapered from the distal end to the proximal end. The ultrasonic tip **17** is in the form of a ball-shaped projection from the end of the ultrasonic probe **16**. This shape of the ultrasonic tip **17** eliminates any sharp edges or surfaces on the tip which could result in damage to tissue during insertion, treatment or removal. The ultrasonic tip **17**, at its distal surface, includes one or more irrigation ports **18**. The irrigation ports **18** are all connected to an internal irrigation passage, preferably centrally located in the ultrasonic tip **17** and the ultrasonic probe **16**. Although not shown in Figure 4, the ultrasonic probe **16** could have, extending along its length, one or more grooves or channels for aspiration, as discussed in more detail below.

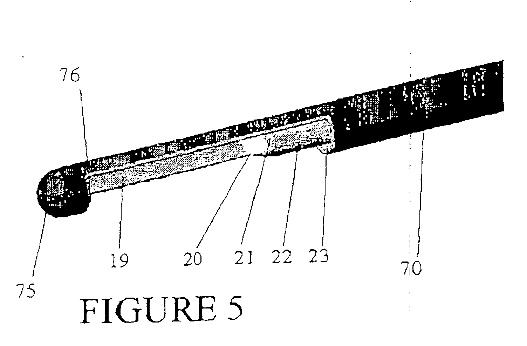

Figure 5 shows a second embodiment of the ultrasonic probe aspiration sheath or catheter of the present invention. The embodiment of Figure 5 is particularly useful for treating conditions wherein the treatment area dangles or is loose; in particular, the embodiment of Figure 5 is useful in gynecological treatments . In the embodiment of Figure 5, the tip **75** of the aspiration sheath or catheter **70** is a rounded end. The aspiration sheath or catheter **70** includes a lateral slot or opening **19** on one side. The ultrasonic probe **23**, with an ultrasonic tip **21** which may include a bevel **20** is mounted for axial sliding movement within the aspiration sheath or catheter **70**. At least one aspiration passage **23** is created in the space between the ultrasonic probe **22** and the interior wall of the aspiration sheath or catheter **70**. Accordingly, as suction is applied to the aspiration fitting or luer **13**, a negative pressure or suction is formed at the aspiration passage **23**, to draw away and destroyed or cavitated tissue and any residual or irrigation fluid.

At the proximal end of the tip **75** is a grasping surface or backstop **76**. This grasping surface or backstop **76** serves as an opposed surface to the ultrasonic tip **21**, thereby allowing dangle or loose treatment areas to be grasped during treatment. In operation, the aspiration sheath or catheter **70** is directed to a treatment area, until the dangling or loose treatment area falls into the lateral slot or opening **19**. During this step, the ultrasonic probe **23** is in a retracted position, as shown in Figure 5. Thereafter, the ultrasonic probe **23** is advanced axially outward, until the dangling or loose treatment area is clamped between the ultrasonic tip **21** and the grasping surface or backstop **76**. Thereafter, the ultrasonic vibration generator is activated, such that ultrasonic energy is transmitted to the ultrasonic tip **21**. As a result, the grasped treatment area is treated using ultrasonic energy and the resulting cavitation.

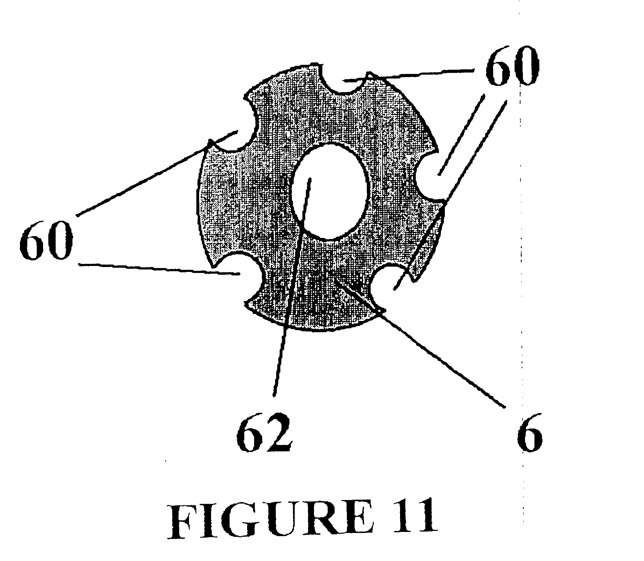

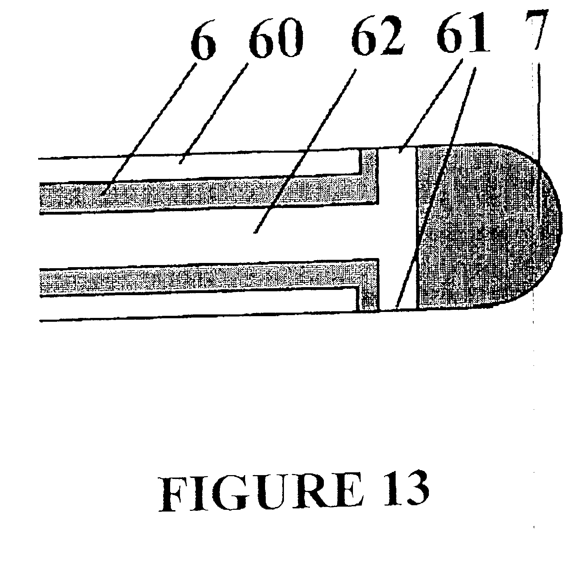

Figures 11 and 13 show a radial cross-section through an ultrasonic probe **6** according to one embodiment of the invention, wherein the probe **6** is particularly useful in urethral treatment of BPH. The probe **6** includes a central passage **62** which is connected to the irrigation fitting or luer **2**. The central passage **62** terminates in two lateral lumens **61**, located on the sides of the probe **6**. The central passage **62** is used to transmit an irrigating fluid to the area around the ultrasonic tip **7**, to thereby regulate the temperature of the treatment site. The irrigation fluid, together with the cavitational action of the ultrasonic tip **7**, allows the treatment site to be regulated to a temperature of ±7°C of normal body temperature. Furthermore, because the lumens **61** do not pass through the ultrasonic tip **7**, the effective area of treatment of;the ultrasonic tip **7** is increased.

As shown in Figures 11 and 13, the outer surface of the ultrasonic probe **6** includes one or more grooves or channels **60**. These grooves or channels, although straight in Figure 13, could spiral along the length of the ultrasonic probe **6**. The grooves or channels **60** are used to aspirate fluid and tissue fragments from the treatment site, as the result of negative pressure or suction applied at the proximal ends of the grooves or channels **60**. As a result, fluid and tissue fragments travel down the grooves or channels **60** and away from the treatment site, thereby preventing fluid and fragments from interfering with the ultrasonic processing and cavitation of additional tissue.

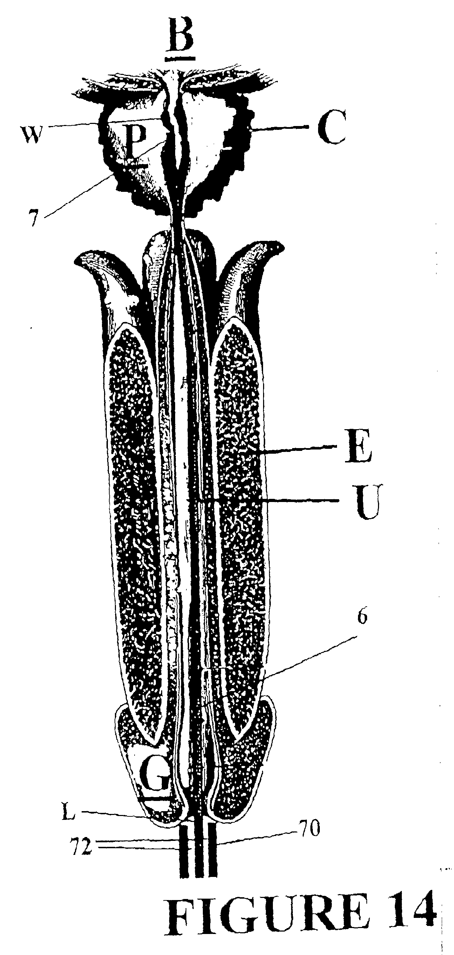

Figure 14 shows the manner in which the ultrasonic probe **6** of Figures 11 and 13 may be used to urethrally debulk prostatic tissue. The ultrasonic probe **6** is inserted into the urethra **U** by inserting it through the lumen **L** of the urethra **U** at the glans **G**. The ultrasonic probe **6** is pushed down the urethra **U** until it is adjacent the urethral walls **W** adjacent the prostate **P** which project into and obstruct the urethra **U**. Next, the aspiration sheath or catheter **70** is slid axially along the length of the ultrasonic probe **6** until its distal end contacts, and seals against, the lumen **L** of the urethra **U** located at the glans **G**. Irrigation fluid may then be supplied to the central passage **62** and negative pressure or suction applied to the aspiration passage **72** which is located between the outer surface of the ultrasonic probe **6** and the inner surface of the aspiration sheath or catheter **70**. Next, the ultrasonic energy vibration source is activated, which transmits ultrasonic energy down the ultrasonic probe **6** so that the ultrasonic tip **7** emits ultrasonic vibratory energy. The ultrasonic tip **7** is pushed against the urethral wall **W**, thereby drilling, by the action of cavitation, a small incision or hole through the urethral wall **W**. The ultrasonic tip **7** is pushed through the hole in the urethral wall **W** until it enters the interior of the prostate **P**. The urethral wall **W** will tend to dilate around the ultrasonic probe **6**, thereby ensuring that the hole is of minimal size. The position of the ultrasonic tip **7** shown in Figure 14 is after the ultrasonic tip **7** has drilled through the urethral wall and has entered into the prostatic tissue. The physician then manipulates the ultrasonic apparatus to move the ultrasonic tip **7** to various areas within the prostate **P**, thereby debulking the prostatic tissue in the immediate vicinity of the ultrasonic tip **7** as the ultrasonic tip **7** moves to different locations in the prostate **P**. As the tissue is being debulked, the suction or negative pressure at the aspiration passage **72** creates a suction or negative pressure in the area of the grooves or channels **60**, thereby drawing fluid and fractured debulked tissue away from the treatment site, down the grooves or channels **60**, into the aspiration passage **72**, through the aspiration fitting or luer **13**, and to a collection or disposal area. Similarly, as the tissue is being debulked, irrigating fluid may be provided through the irrigation fitting or luer **2**, down the central passage **62,** and out the lumens **61**, to thereby regulate the temperature at the treatment site and to provide a vehicle for transmission of the fragments down the grooves or channels **60**.

Once a cavitated lesion of sufficient size has been created, the physician will moves the ultrasonic probe **6** to the next site to be reduced and repeats the process. It is anticipated that in a preferred method of treatment of the present invention, two or more cavitational treatments in each lateral prostatic lobe would be performed - depending upon the size of the prostate **P** - thereby reducing the size of the prostate **P** and resolving the urethral obstruction. The divergent water content and compliancy of other urethral tissues, such as the urethral wall **W** adjacent to the prostatic lobes being treated protects the prostatic capsule **C**, bladder **B** neck and sphincter from any residual damage, as the ultrasonic energy results in very localized cavitation damage in a small area around the ultrasonic tip **7**, thereby eliminating residual damage which is attendant to less localized treatment methods. When the ultrasonic probe **6** is withdrawn through the hole in the urethral wall **W**, the urethral wall **W** will dilate, so as to seal or substantially seal the hole.

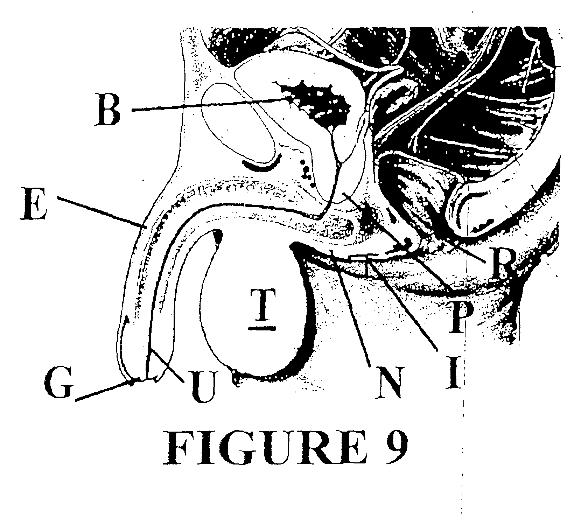

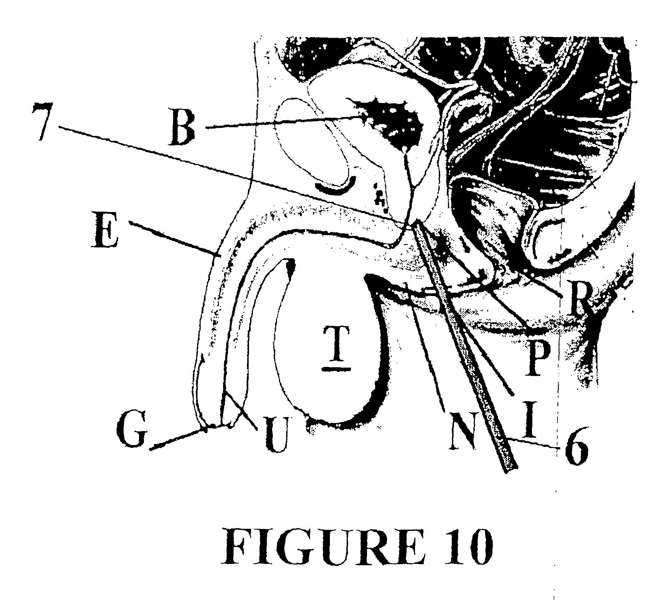

Figures 9 and 10 show a perineal treatment method of the present invention. Figure 9 shows a cross-sectional view of the lower portion of a male patient. The perineum **N** is the area in the male between the scrotum (which holds the testes **T**) and the rectum **R** The perineum **N** provides a short path for access to the prostate **P**. In the perineal method of the present invention, the patient is placed supine on an operating table, and a local or general anesthetic is administered. A small incision **I** is made in the perineum **N**, as shown in Figure 9. Thereafter, the ultrasonic probe **6** is inserted into the perineal incision, and the ultrasonic tip **7** is activated. Ultrasonic energy at the ultrasonic tip **7** is used to drill through the prostatic capsule **C** and thereafter the ultrasonic tip **6** is inserted, and moved within, the prostate **P**, thereby debulking prostatic tissue within the prostate **P** immediately adjacent the ultrasonic tip **7**. Aspiration and irrigation are applied in a manner identical to the manner discussed above with reference to the urethral treatment method, with the distal end of the aspiration sheath or catheter **70** being placed against the perineum at the location of the incision **I**. After each prostatic lobe has been treated in this manner, the ultrasonic probe **6** is withdrawn, and the incision **I** is closed using known surgical techniques.

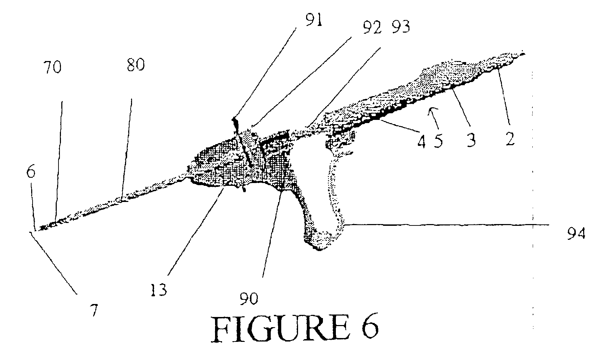

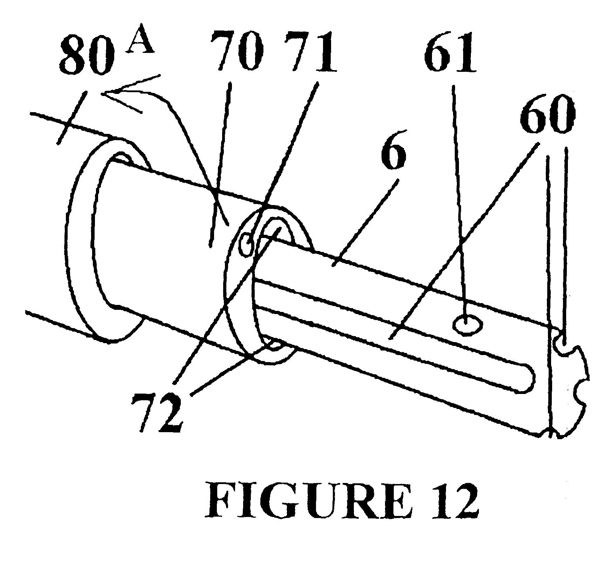

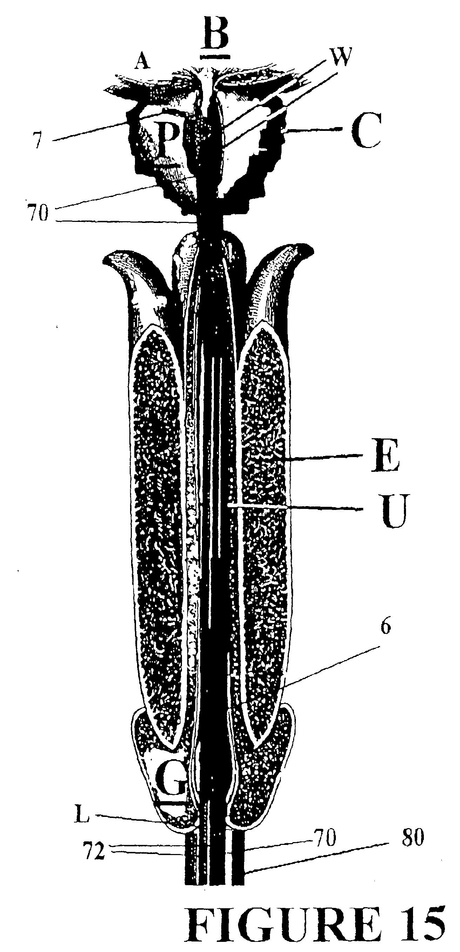

Figures 6 and 12 show features of an ultrasonic treatment apparatus of another embodiment of the present invention, and Figure 15 shows the manner in which that embodiment is used in a urethral prostate debulking procedure. As shown in Figure 6, the ultrasonic treatment apparatus has an ultrasonic probe **6** with an ultrasonic tip **7**. The ultrasonic probe **6** is housed in, for slidable movement within, a flexible articulation sheath **70**. The flexible articulation sheath **70** is, in turn, housed in for slidable movement within, a rigid sheath **80**. Rigid sheath **80** is connected to, for movement with, a retracting housing **90**. The retracting housing **90** is connected to a retracting trigger **94**, which is pivoted on the handle **5**. The retracting housing **90** may include an aspiration fitting or luer **13**, which is configured for connection with a flexible tube which is in turn connected to a source of reduced pressure. As discussed in more detail below, the aspiration fitting or luer **13** is connected to the interior ofthe flexible articulation sheath **70**.

An articulation trigger **91** may be housed on the retracting housing **90**. Articulation trigger **91** is connected to an articulation wire **71** discussed in more detail below. A trigger **92** may also be housed on the retracting housing **90**. A cover **93** may cover components between the retracting housing **90** and the handle **5**.

Figure 12 shows the details of the proximal end of the ultrasonic apparatus of Figure 6. The ultrasonic probe **6** may include one or more grooves or channels **60** which are used to provide aspiration to the area around the ultrasonic tip **7**. One or more irrigation lumens **61** may provide irrigating fluid to the area around the ultrasonic tip **7**. The ultrasonic probe **6**, which, because of its small cross-sectional profile and the material of which it is constructed, is somewhat flexible so that it may be bent or articulated. The ultrasonic probe **6** fits within, for axial movement, the articulation sheath **70**, which is made of a relatively flexible and resilient material. The space **72** between the ultrasonic probe **6** and the articulation sheath **70**, together with the grooves or channels **60**, form aspiration passages. The articulation sheath **70** may include, at one or more locations around the circumference of the articulation shaft **70**, one or more embedded articulation wires **71**, with a distal end affixed to the articulation sheath **70**. The proximal end of the articulation wire **71** is affixed to the articulation trigger **91**. The articulation sheath **70** is housed within, for axial ; movement, the rigid sheath **80**. Rigid sheath **80** is made of a relatively rigid material.

When the rigid sheath **80** is slid back away from the distal end of the articulation sheath **70**, and the articulation wire **71** is pulled axially inwardly by the articulation trigger **91**, the articulation sheath will bend or articulate in a bending or articulation direction **A**. As a result, the ultrasonic probe **6** and ultrasonic tip **7** will bend or articulate in articulation direction **A**. In this way, the ultrasonic can be used to reach locations which are not axially aligned with the lumen or vessel through which the ultrasonic probe **6** is inserted.

Figure 15 shows the manner in which the embodiment shown in Figures 6 and 12 may be used to debulk prostatic tissue. In a first step, the rigid sheath **80**, articulation sheath **70** and ultrasonic probe **6** are all in an extended position, so that the distal ends of each are approximately adjacent to one another. The rigid sheath **80**, articulation sheath **70** and ultrasonic probe **6** are then all advanced into the patient's urethra **U**, until the ultrasonic tip **7** is in the area of the urethral wall **W** adjacent the bulked prostatic lobe of the prostate **P**. Next, the retracting trigger **94** is actuated, such that the rigid sheath **80** is withdrawn from the urethra **U**, to a location outside of the lumen **L** of the urethra **U** (as shown in Figure 15). The distal end of the articulation sheath **70** is thereafter bent or articulated in a direction **A**, by activating the articulation trigger **91** such that it pulls the proximal end of the articulation wire **71** axially inwardly. This bending or articulation of the articulation sheath **70** in turn acts to bend or articulate the distal end of the ultrasonic probe **6**. Thereafter, the ultrasonic probe **6** may be advanced axially outwardly, so that it may ultrasonically drill through the urethral wall **W** and into the prostate **P** (as shown in Figure 15), where it may be used to ultrasonically debulk prostatic tissue.

In a preferred embodiment of the invention, maximum vibratory motion is not confined to the tip of the probe as in the case of prior art ultrasonic instruments. Rather, the probe of the invention is specially designed to provide a multiplicity of so called anti-nodes (i.e., points along the probe where maximum vibration occur) at spaced intervals along the axial length of the probe, in addition to the tip of the probe. This construction best suits the method of the invention because removal of tissue will not be confined to those regions of the tissue coming into contact with the tip of the probe. Rather, as the probe is swept through the tissue, preferably in a windshield-wiper fashion as described above, the tissue is removed in all areas adjacent to the multiplicity of anti-nodes located along the entire length of the probe. In this way, the apparatus of the invention allows for tissue removal in accordance with the method of the invention to be carried out most efficiently so that actual treatment time is greatly reduced as compared to prior art methods.

Furthermore, the mode of vibration of the ultrasound probe in the apparatus of the invention differs from the axial mode of vibration which is conventional in the prior art. Rather than vibrating exclusively in the axial direction, the probe in the apparatus of the present invention vibrates in a direction transverse to the axial direction. Because of this transverse mode of vibration, the probe of the invention removes tissue not just at those points where the probe makes actual contact with the tissue, but also typically in a region having a radius up to 1.0 - 1.5 mm around the probe Hence, the transverse mode of vibration of the probe used in the present apparatus also contributes to the efficiency of the method of the invention by expanding the coverage area around the probe where tissue is removed.

In general, in order to increase the number of anti-nodes occurring along the axial length of the probe, the vibration frequency imparted to the probe should be increased. The frequency, however, is not critical and a generator run at 20 kHz is generally sufficient to provide for an effective number of anti-nodes along the axial length of the probe. In addition, as will be appreciated by those skilled in the art, it is possible to adjust the dimensions of the probe, including diameter, length and location of coupling to the ultrasonic energy source, in order to space the anti-nodes at desired intervals. Applicant's co-pending application Ser. No. 60/178,901 further describes the design parameters for an ultrasonic probe operating in a transverse mode, and is herein incorporated by reference.

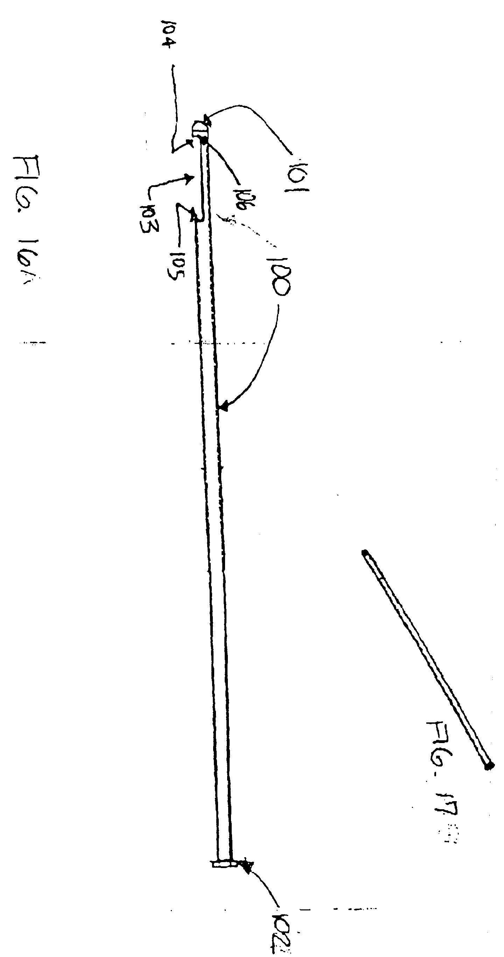

FIGS. 16 and 17 show an alternative ultrasonic tip for use with the current invention. The tip 100 is generally cylindrical with a distal end 101 and a proximal end 102. Near the distal end of the tip there is a recessed portion 103 with a distal end 104 and a proximal end 105. The recessed portion 103 is bounded at the distal end 104 with a planar surface 106. The planar surface is positioned at a 90° angle to the longitudinal axis shaft of the ultrasonic tip. The proximal end 102 of the tip is adapted to be connected to an ultrasonic vibration source (not shown). The probe is constructed from any suitable titanium alloy. Upon application of ultrasonic vibration, the vibrations are transmitted to the planar surface. The entire area of the planar surface then acts as a cutting surface. An operator of the ultrasonic probe can then utilize the probe for resections similar to that of standard electrocauter resections, but without the thermal effect.

Thus, there is shown and described a unique design and concept of an ultrasonic treatment device and method of its use. While this description is directed to particular embodiments, it is understood that those skilled in the art may conceive modifications and/or variations to the specific embodiments shown and described herein. Any such modifications or variations which fall within the purview of this description are intended to be included as part of the invention. It is understood that the description herein is intended to be illustrative only and is not intended to be limitative. Rather, the scope of the invention described herein is limited only by the claims.

## Claims

1. An ultrasonic treatment apparatus comprising:
an ultrasonic probe having an ultrasonic tip; and
an aspiration sheath surrounding the ultrasonic probe, the aspiration sheath forming at a distal end an aspiration port, the aspiration sheath being moveable axially relative to the ultrasonic probe.

2. The ultrasonic treatment apparatus of claim 1 wherein:
the ultrasonic probe includes at least one channel on an outer surface of the ultrasonic probe, the at least one channel extending from a proximal end of the ultrasonic probe to a location adjacent the ultrasonic tip.

3. The ultrasonic treatment apparatus of claim 2 wherein:
the aspiration port communicates with the at least one channel.

4. The ultrasonic treatment apparatus of claim 2 or claim 3 wherein:
the ultrasonic probe includes a plurality of channels.

5. The ultrasonic treatment apparatus of any one of claims 2 to 4 wherein:
the at least one channel spirals around the outer surface of the ultrasonic probe.

6. The ultrasonic treatment apparatus of any preceding claim wherein:
the ultrasonic probe includes an irrigation passage.

7. The ultrasonic treatment apparatus of claim 6 wherein:
the irrigation passage is centrally located in the ultrasonic probe.

8. The ultrasonic treatment apparatus of claim 6 or claim 7 wherein:
the irrigation passage includes at least one lumen on a side of the ultrasonic probe.

9. The ultrasonic treatment apparatus of any preceding claim further comprising:
a flexible fiberoptic viewing device attached to the aspiration sheath.

10. The ultrasonic treatment apparatus of any preceding claim wherein:
the aspiration sheath is formed of a flexible and resilient material and includes articulation wire so that the aspiration sheath may be controllably articulated.

11. The ultrasonic treatment apparatus of claim 10 wherein:
when the aspiration sheath is controllably articulated, the probe is deflected.

12. The ultrasonic treatment device of any preceding claim wherein:
the aspiration port is a lateral slot located on one side of the aspiration sheath.

13. An ultrasonic treatment apparatus comprising:
an ultrasonic probe having an ultrasonic tip, the ultrasonic probe including as least one channel on an outer surface of the ultrasonic probe, the at least one channel extending from a proximal end of the ultrasonic probe to a location adjacent the ultrasonic tip.

14. The ultrasonic treatment apparatus of claim 13 wherein:
the ultrasonic probe includes a plurality of channels.

15. The ultrasonic treatment apparatus of claim 13 or claim 14 wherein:
the at least one channel spirals around the outer surface of the ultrasonic probe.

16. The ultrasonic treatment apparatus of any one of claims 13 to 15 further comprising:
an aspiration sheath surrounding the ultrasonic probe, the aspiration sheath forming at a distal end an aspiration port, the aspiration port communicating with the at least one channel.

17. The ultrasonic treatment apparatus of claim 16 wherein:
the aspiration sheath os moveable axially relative to the ultrasonic probe.

18. The ultrasonic treatment apparatus of claim 16 or claim 17 wherein:
the aspiration sheath is formed of a flexible and resilient material and includes an articulation wire so that the aspiration sheath may be controllably articulated.

19. The ultrasonic treatment apparatus of claim 18 wherein:
the aspiration sheath can be used to bend the ultrasonic probe.

20. The ultrasonic treatment apparatus of any of claims 15 to 19 wherein:
the aspiration probe includes an irrigation passage.

21. The ultrasonic treatment apparatus of claim 20 wherein:
the irrigation passage is centrally located in the ultrasonic probe.

22. The ultrasonic treatment apparatus of claim 21 or claim 22 wherein:
the irrigation passage includes at least one lumen on a side of the ultrasonic probe.

23. The ultrasonic treatment apparatus of any of claims 13 to 22 further comprising:
a flexible fiberoptic viewing device attached to the probe.

24. The ultrasonic treatment apparatus of any of claims 13 to 23 wherein:
the aspiration port is a lateral slot located on one side of the aspiration sheath.

25. An ultrasonic probe comprising:
an elongate shaft with a longitudinal axis with a recessed portion, the recessed portion bounded at one end with a planar surface.

26. The ultrasonic probe of claim 25 wherein the planar surface is approximately 90° to the longitudinal axis of the elongate shaft.
